# EUROPEAN PATENT SPECIFICATION

(11) **EP 1 399 095 B1**
(45) Date of publication and mention of the grant of the patent: **21.06.2017**
(21) Application number: 02743581.7
(22) Date of filing: 27.06.2002
(51) Int. Cl.: A61F 2/06

(54) **A CATHETER**
KATHETER
CATHETER

(30) Priority: 27.06.2001 IE 20010591; 02.07.2001 US 301820 P
(43) Date of publication of application: 24.03.2004
(73) Proprietor: Salviac Limited, Dublin 2 (IE)
(72) Inventor: BRADY, Eamon, Elphin, County Roscommon (IE); FARRELL, Fergal, Moone, Athy, County Kildare (IE)
(74) Representative: O'Brien, John Augustine
(86) International application number: PCT/IE2002/000092
(87) International publication number: WO 2003/002033

(56) References cited:
- WO-A-00/69499

## Description

### Introduction

Many intravascular catheter designs require that one tube move relative to another. Typically an outer tube may provide a reception space for a medical device during delivery or retrieval. The inner tube may provide a deployment function, or a centering function. Examples of such catheters include self-expanding stent delivery systems, filter delivery systems and retrieval catheters. In all these cases mechanical forces are transmitted from one end of the tube to the other. These forces may be tensile (pull), or compressive (push). Typically when a pull force is applied to one of the tubes, a reaction push force is set up in the other and relative movement takes place (and vice versa). Typically a guidewire lumen is provided through the center of the inner tube.

Many catheter systems are provided in rapid exchange format as these systems are considered to be easier to use by many clinicians. This requires that the guidewire travel from the inner lumen through the wall of the inner tube and subsequently through the wall of the outer tube. The catheter construction must allow relative movement of the inner and outer tubes in this area. This problem is typically solved by providing a slot in the wall of either the inner of the outer and an exit port in the wall of the other. The slot must be at least the length of the longest anticipated stroke of the inner relative to the outer (or vice versa).

Such systems have a number of serious limitations associated with the slot. Firstly, the presence of a slot in the shaft significantly compromises the mechanical integrity of the shaft. Slots cut into the walls of tubes are very prone to kinking, are unable to resist torque loading and often have a tendency to collapse inwardly. The reduction in mechanical properties is contributed to in part by the reduction in cross section but also by the lack of self-support that is integral to a tube. These problems are particularly evident when the wall of the tube is thin.

In general the guidewire exit port location of catheters for rapid exchange use in a transition zone where the catheter is prone to kinking and damage due to a non-linear transition from the distal to proximal section of the catheter. The incorporation of a slot also affects the overall symmetry of the catheter and therefore will generally be the location where any unwanted deformation will be initiated.

There is therefore a need for an improved construction of catheter which will overcome at least some of these problems.

US-A-5,690,644 describes a stent delivery system of the type comprising:
a catheter including an elongate shaft;
the elongate shaft having a proximal end, a distal end and a wall defining a shaft lumen; and
an inner member extending through the shaft lumen.

### Statements of Invention

The invention is characterised in that the inner member is a solid rod member, in that the solid rod member has a surface pathway defined by a longitudinally extending partially open portion of the surface of the solid rod member and extending along the length of the solid rod member (3). to direct a guidewire from a distal guidewire port.

In this specification the inner member or pusher is defined as being solid if the guidewire pathway is defined external to the body of the member, and the sum of its cross sectional area and the cross sectional area of the guidewire are substantially equal to the cross sectional area of a rod of the same maximum diameter as the member.

In one embodiment of the invention the proximal guidewire port is positioned a substantial distance distal of the proximal end of the shaft. Preferably the guidewire pathway is offset from a longitudinal axis of at least a portion of the elongate shaft.

The guidewire pathway may be partially defined by an inner surface of the elongate shaft.

In one case the solid member is slideably translatable relative to the elongate shaft. In another case the solid member is slideably translatable relative to a guidewire.

The catheter preferably comprises a reception space at the distal end of the shaft. Ideally the reception space is offset relative to the shaft of the catheter. The reception space may be a pod. Preferably the pod is thin walled. Most preferably the pod is reinforced.

In another embodiment of the invention the inner solid member is reinforced. The reinforcement may be a metal. Preferably the metal is at least partially comprised of one or more of titanium, vanadium, molybdenum, iron, chromium and nickel.

The reinforcement may comprise at least one wire. Preferably the wire is of stainless steel.

The reinforcement may be a fiber. Preferably the fiber is glass, Kevlar, graphite, carbon or a polymeric fiber.

In one case the reinforcement is disposed coaxial to the center of area of the solid member.

Desirably the solid member comprises an abutment surface at its distal end. Preferably the abutment surface is engagable with a medical device for deployment of the device.

In a preferred embodiment a tip is provided at the distal end of the inner member. The tip may be integral with the inner member. Preferably the tip provides a smooth transition between the guidewire and the catheter shaft. Ideally the tip defines a pathway through the medical device. Most preferably the tip is of a soft atraumatic material.

The lumen of a medical device in the reception space is preferably interfaced with the guidewire surface pathway of the solid member. In one case the interface comprises a ramp feature. In another case the interface comprises a portion of a funnel.

Ideally the cross sectional area of the inner member added to the cross sectional area of the guidewire comprise substantially the cross sectional area of the inner lumen of the shaft.

In another embodiment of the invention a marker band is provided at the distal end of the inner solid shaft. The marker band may be attached to the inner shaft. Preferably the marker band is solid in construction.

In a further case the solid member is rotationally fixed relative to the elongate shaft. The system may comprise a key to prevent rotational movement. In one embodiment the key is provided in a portion of the outer shaft. In another embodiment the key is provided at the guidewire exit port. Preferably a ramp is provided at the exit port and the ramp defines the key. In a further embodiment the key is provided in a handle portion of the shaft.

In a particularly preferred embodiment the guidewire exit port comprises an opening in the wall of the elongate shaft. Preferably the system comprises a ramp for guiding a guidewire to the exit port. Ideally the ramp comprises a tongue-like segment extending inwardly from the exit port. Most preferably the tongue extends into the surface guidewire pathway in the inner member. The tongue may substantially fill the cross section of the surface guidewire pathway. Preferably the ramp surface has a cylindrical aspect. The tip may be offset relative to the guidewire.

The exit port is preferably sized so as to provide clearance to the guidewire.

In one case the guidewire exit port is generally angulated in the direction of guidewire movement.

In one embodiment of the invention the catheter is a filter delivery catheter. Preferably the shaft has a distal pod defining a reception space for a filter.

In another embodiment the catheter is a stent delivery catheter. Preferably the shaft has a reception space at the distal end for reception of a stent. Most preferably the inner member extends through a lumen defined by the stent. Ideally the inner member terminates in a distal tip.

In a further embodiment the catheter is a filter retrieval catheter. Preferably the shaft has a distal reception space for reception of a retrieved filter. The inner member may extend through the reception space during positioning of the catheter for filter retrieval. Preferably the inner member projects distally of the distal end of the shaft to provide centring.

These and other problems are solved by the catheter designs of this invention. Rapid exchange catheters are provided that deliver all of the advantages of a dual tube systems without the drawbacks of these systems when configured in a rapid exchange slot and hole format.

The designs of this invention provide a solid inner member and an outer tube with a discrete exit port. A guidewire surface pathway is provided that guides the guidewire to the exit port. A variety of exit port configurations are possible.

The fact that the inner member is solid provides a range of advantages not available with tubular systems. Firstly, the inner member maintains cross-sectional area over its entire length. This ensures that it has no mechanical weak points and is homogenous in its properties over its length. Solid shafts are not susceptible to kinking. Solid shafts provide excellent push and tensile transmission. Solid shafts have lower bending stiffness than tubes of the same cross-sectional area due to their lower second moment of area.

The catheters of this invention allow relative movement between an outer catheter and inner pusher without the inclusion of a slot in either the inner or outer elements. A guidewire rapid exchange exit port is included in the outer catheter. This exit port is sized to accommodate the exit of the guidewire. The exit port preferably has a tapering aspect to minimize the force required to manipulate the guidewire out through the exit port.

A very important feature of the inner members of the invention is the fact that the material volume is concentrated close to the neutral axis of the inner member. This provides an excellent relationship between the push and trackability properties of the inner member. Push properties tend to be dominated by the cross sectional area of the member and the material properties. Trackability properties depend strongly on the distance the material is spread from the neutral axis (2^{nd} moment of area) and the material properties. Solid members are advantageous because the presence of a lumen in the center of a member results in material being distributed farther from the neutral axis.

Another advantage is derived from the fact that the surface area available for friction build up is minimized. Tubular systems have the disadvantage that movement of the inner relative to the outer and the guidewire generates significant frictional forces. This situation arises because the inner has a large surface area when its inner and outer surfaces are added together. This large surface area adds to the drag when the inner element is moved relative to the other parts of the system. Tubular systems also build up frictional forces with the guidewire. When the system is placed in a tortuous vessel the bending forces of the guidewire are applied directly to the surface of the inner. This sets up normal forces on the inner that do not affect the inner members of this invention.

Because the inner members of this invention are solid no inner frictional surface exists. Further more normal forces associated with the bending stiffness of the guidewire are not transmitted to the inner member since the guidewire is accommodated on the outer surface of the inner member.

The transmission of mechanical forces can be further optimized with this invention through the use of reinforcements. Because the guidewire has been moved from the central axis location it is now possible to incorporate reinforcing wires or fibers or members in that location. Reinforcements work particularly well with this invention as they can be placed very close to the neutral axis of the inner member irrespective of its plane of bending. Tubular elements require that reinforcements be placed in the walls of the tube. When push properties are desired metallic reinforcements such as wires are desirable. Solid wires or bundles of wires are excellent for transmitting push. Braided systems can be used to optimize torque.

Regardless of the type of reinforcement employed it is desirable that it be placed as close to the center of area of the cross section of the member as possible. Where it is not placed exactly on the center of area it should be centered over the center of area. It should be noted that the center of area is not the same as the central axis of the member. The presence of the surface guidewire pathway shifts the center of area very slightly. Centering reinforcements around the center of area ensures that the properties of the reinforced shaft are very homogenous.

When crossing a lesion with a device it is often advantageous to have the guidewire centrally located within the device and, in particular, the distal portion of the device which crosses the lesion first. The use of surface guidewire pathways can result in the guidewire being off center relative to outer shaft. In many situations this feature presents no difficulty and may even be advantageous to the manoeuvring of the catheter. Where it is required to maintain the guidewire central to the medical device at the distal end a number of guiding features may be provided.

The reception space at the distal end of the shaft may be offset relative to the shaft so as to ensure the alignment of the surface guidewire pathway with the central lumen of the medical device.

In another variation the distal end of the inner member may be provided with a discrete ramp that directs the wire from the central axis of the medical device to the surface guidewire pathway. A variety of ramp configurations are possible including a funnel, a ramp with a circular aspect or a tunnel style ramp.

A significant advantage of this invention is that relative movement of the inner member and the outer catheter does not affect the rapid exchange capability in any way. The surface pathway provided in the inner ensures that the guidewire is in contact with the surface of the outer. This ensures that only small lateral movement is required to direct the guidewire to exit the exit port. This feature ensures that the guidewire movement of the catheter is good. The orientation of the surface guidewire pathway is maintained relative to the exit port so as to ensure that the end of the guidewire is presented to the exit port in the correct orientation when the catheter is being loaded onto a guidewire. This orientation needs to be maintained only in the area of the exit port. A number of features are provided per this invention to ensure that orientation is maintained.

In one embodiment a keying arrangement is provided in the proximal section of the catheter. In one variation this key is provided on the inner surface of the outer and engages the surface pathway proximal to the exit port. This key may also be used to provide an exit ramp at the exit port.

In another variation the exit port ramp feature engages the surface guidewire pathway and prevents relative rotational movement of the inner and outer.

In yet another arrangement the keying function is located in the handle area. This system has the advantage that the user cannot attempt to rotate one element relative to the others and is preferred for this reason. Indeed, more than one keying feature can be used simultaneously.

An important feature of all these systems is that they do not inhibit longitudinal movement of the two members.

### Brief Description of the Drawings

The invention will be more clearly understood from the following description thereof given by way of example only, in which:-
Fig. 1 is an isometric view of a portion of a catheter according to the invention;
Fig. 2 is a cross sectional view on the plane A of the catheter of Fig. 1;.
Fig. 3 is a cross sectional view on the plane B of the catheter of Fig. 1;.
Fig. 4 is a perspective view of the catheter with a filter in a pre-deployment or delivery configuration;
Fig. 5 is a perspective view of the catheter with a filter in a deployed configuration;
Fig. 6(a) is a cross sectional view of a vessel with a lesion which has been crossed with a guidewire;
Fig. 6(b) illustrates a delivery catheter containing a filter which has been passed over a guidewire to an intended vascular site ready for deployment;
Fig. 6(c) illustrates a partially deployed filter exiting the catheter at the deployment site;
Fig. 6(d) illustrates the filter in its deployed state at an intended site distal to a lesion;
Fig. 6(e) illustrates a filter deployed on a guidewire distal to a lesion site;
Fig. 7 and 8 are detailed cross-sectional views of a rapid exchange exit port region of the catheter;
Fig. 9 is a cross-sectional view on the line A of Fig. 8;
Fig. 10 is a cross-sectional view on the line A1 of Fig. 8;
Fig. 11 is a cross-sectional view of a distal end of the catheter showing a ramp that directs a guidewire from the central axis of a medical device and along a guidewire surface path;
Fig. 12 is a cross-sectional view of a distal end of a catheter showing a guidewire extending along a guidewire surface path;
Fig. 13 is a cross-sectional view of a detail of a catheter showing a keying arrangement provided in the proximal section of the catheter, a key being provided on the inner surface of the outer and engaging a surface pathway proximal to the exit port. This key may also be used to provide an exit ramp at the exit port;
Fig. 14 and 15 are cross-sectional views of a detail of a catheter showing an inwardly directed tongue on the outer catheter, the tongue engaging a surface pathway proximal to the exit port, this tongue may also be used to provide an exit ramp at the exit port;
Fig. 16(a) is a cross-sectional view of a catheter containing a stent in a pre-deployment or delivery configuration;
Fig. 16(b) is a cross-sectional view of the catheter of Fig. 16(a) with a stent in a deployed configuration;
Fig. 17(a) is a cross-sectional view of another catheter containing a stent device in a pre-deployment or delivery configuration;
Fig. 17(b) is a cross-sectional view of the catheter of Fig. 17(a) with a stent in a deployed configuration;
Fig. 18(a) is a cross-sectional view of a filter retrieval catheter in a pre-retrieval configuration;
Fig. 18(b) is a cross-sectional view of the catheter of Fig. 18(a) with a filter in a retrieved configuration;
Fig. 19 is a cross-sectional view of a solid inner pusher containing a solid wire at its neutral axis;
Fig. 20 is a cross-sectional view of a solid inner pusher containing a multi-filament wire at its neutral axis;
Fig. 21 is a cross-sectional view of a co-extruded solid inner pusher;
Fig. 22 is a cross-sectional view of an inner pusher as a solid member.

### Detailed Description

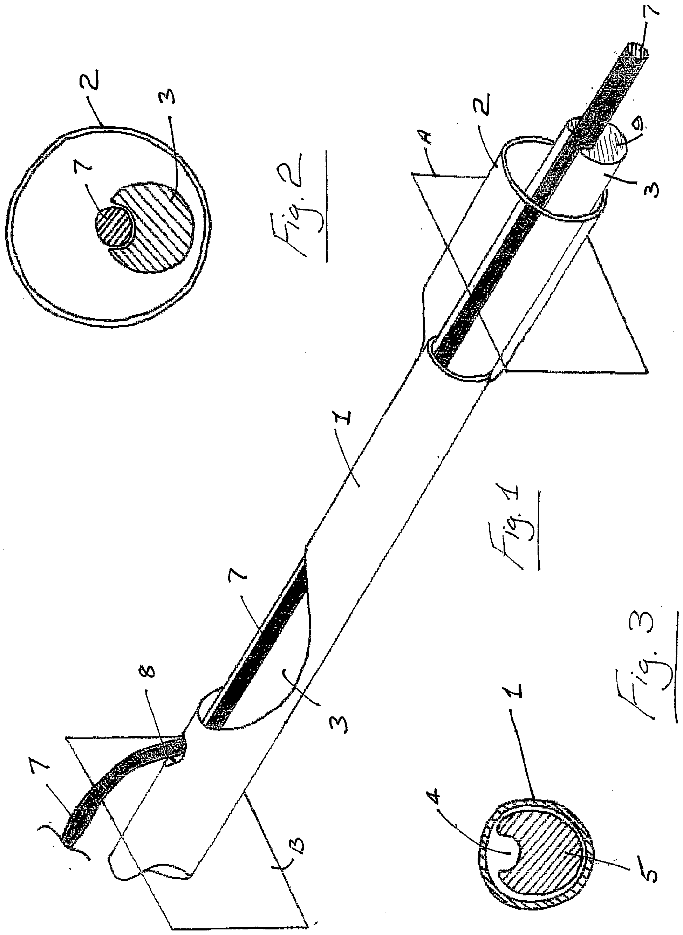

Referring to Figs. 1 to 3 there is illustrated a catheter suitable for the delivery of a medical device such as a stent or a filter. The catheter has an outer shaft 1 with a distal end and a proximal end. The distal end comprises a pod 2 that provides a reception space for a medical device. The catheter has an inner shaft 3 comprising a solid rod 5, a surface guidewire pathway 4 for a guidewire 7. The inner shaft defines an abutment surface 9 at its distal end. The guidewire pathway 4 extends longitudinally and is configured so as to accommodate the guidewire 7. The abutment surface 9 from the pod 2 is configured so as to engage with a medical device to achieve deployment of the medical device from the pod 2. In Figs. 1 to 4 the catheter is shown in a deployed configuration. In this deployed configuration the distal end of the inner shaft 3 is in an advanced position relative to the pod 2. The outer shaft 1 further comprises an exit port 8 where the guidewire exits the catheter in a rapid exchange manner. It will be noticed that the pod 2 is shown offset relative to the shaft 1. This off set feature ensures that the central axis of the medical device is in alignment with the surface guidewire pathway 4.

Fig 2 shows a cross sectional view of the catheter in the region of the pod 2. This view shows the guidewire 7 located in the pathway 4 of the inner shaft 3. It will be noticed however that the guidewire 7 is positioned centrally relative to the pod 2. This is achieved through the offset aspect of the pod 2 relative to the shaft 1.

Fig. 3 shows a cross sectional view of the catheter proximal to the exit port 8. The outer shaft is shown in a concentric arrangement with the solid inner shaft 5.

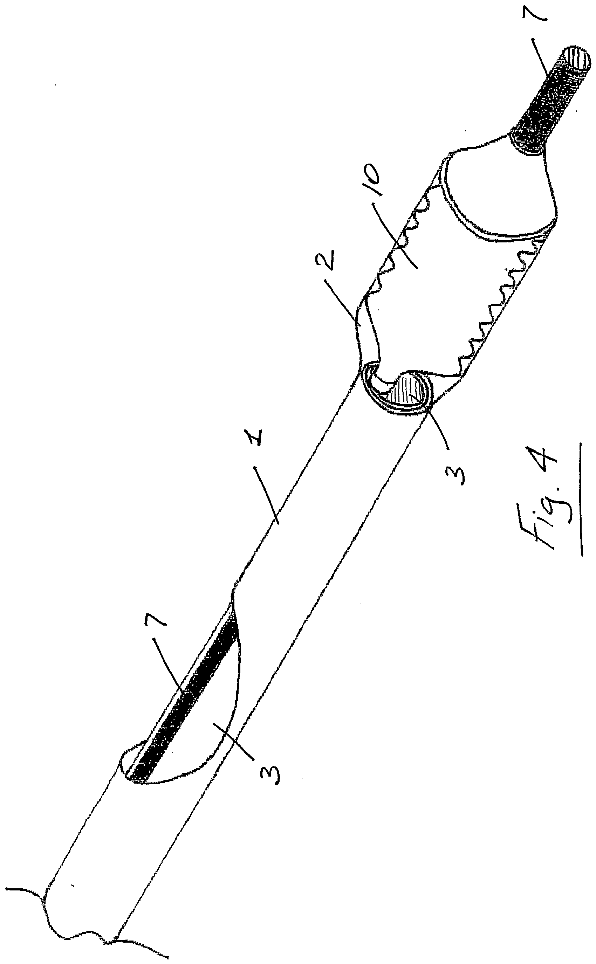

An isometric view of the rapid exchange technology of the invention is shown applied to a filter delivery system in Fig. 4. The guidewire 7 is shown protruding from the distal end of a filter 10. The filter 10 is in a collapsed or wrapped down configuration in the pod 2. A proximal end of the filter 10 is in abutment with the distal end of the inner member 3 such that a guidewire lumen of the filter is in alignment with the guidewire surface pathway 4. The offset pod 2 facilitates this alignment.

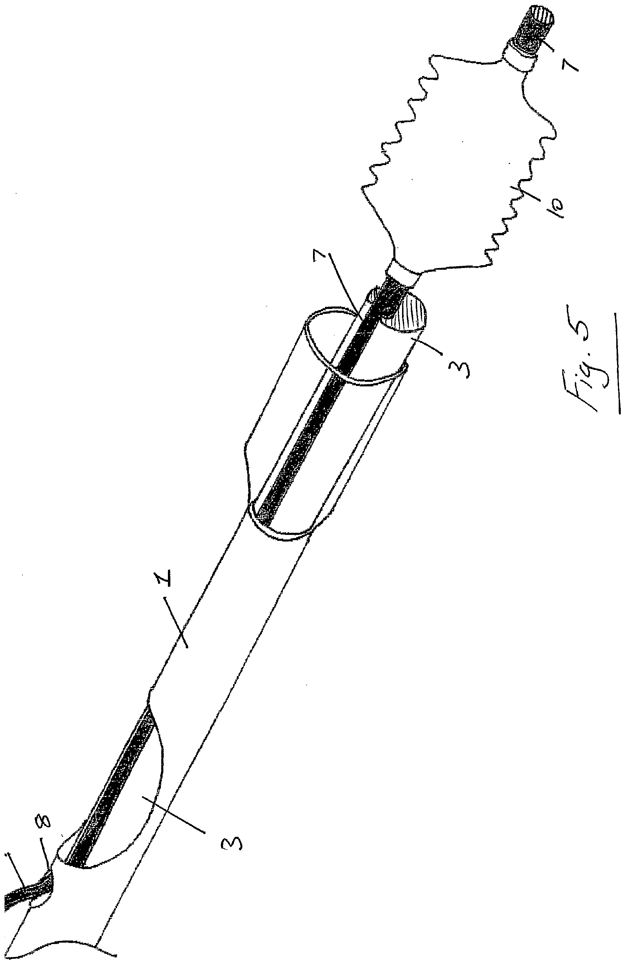

Another isometric view of the rapid exchange filter delivery system is shown in Fig 5 in which the system is illustrated with the filter 10 in the deployed configuration. The inner shaft member 3 is in its advanced position and in so advancing the filter 10 is deployed. Alternatively, the catheter shaft and pod can be retracted to achieve filter delivery. It will be apparent that the rapid exchange filter delivery system of this invention can be applied to filter systems that are fixed on the wire or to filter systems wherein the filter can move independently of the wire.

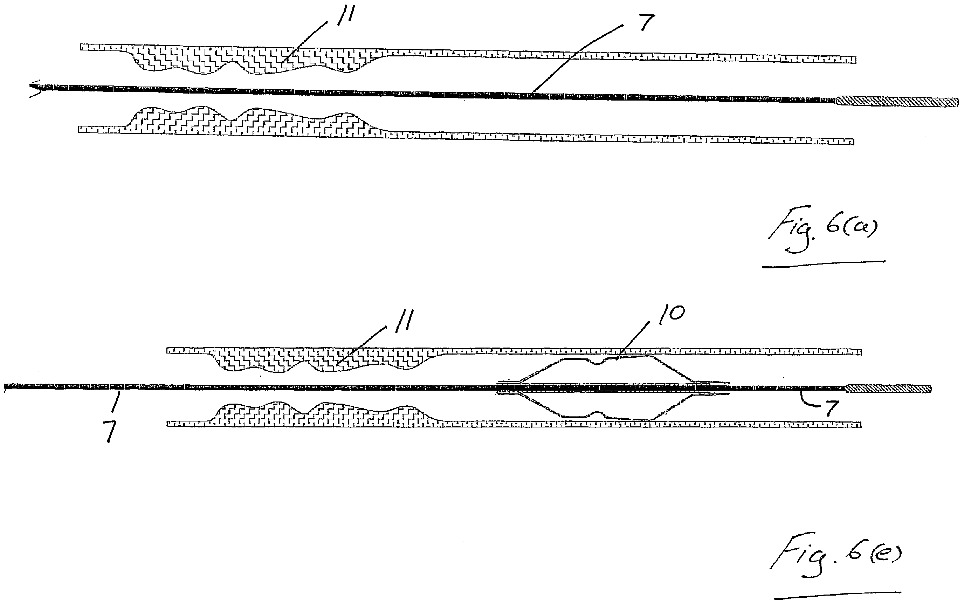

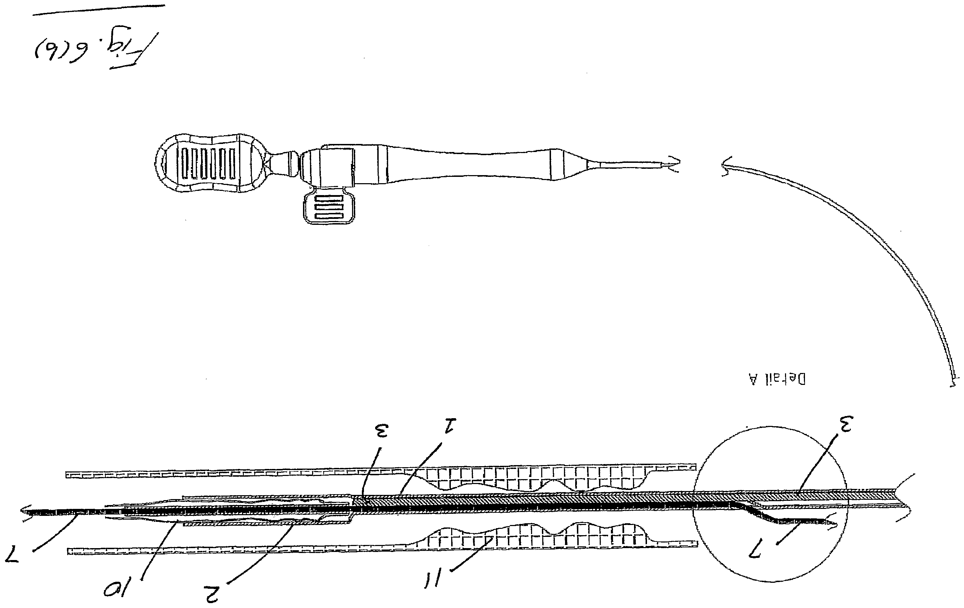

Fig. 6a shows a vascular lesion 11 in a vessel which has been crossed with a guidewire 7. The guidewire 7 is in a position to receive a catheter, as illustrated in Fig. 6b. This schematic illustration shows a catheter according to the invention which contains a filter 10 in a wrapped down configuration in the pod 2. The catheter is shown having tracked over the guidewire 7 and at a position distal to a lesion 11 ready for deployment.

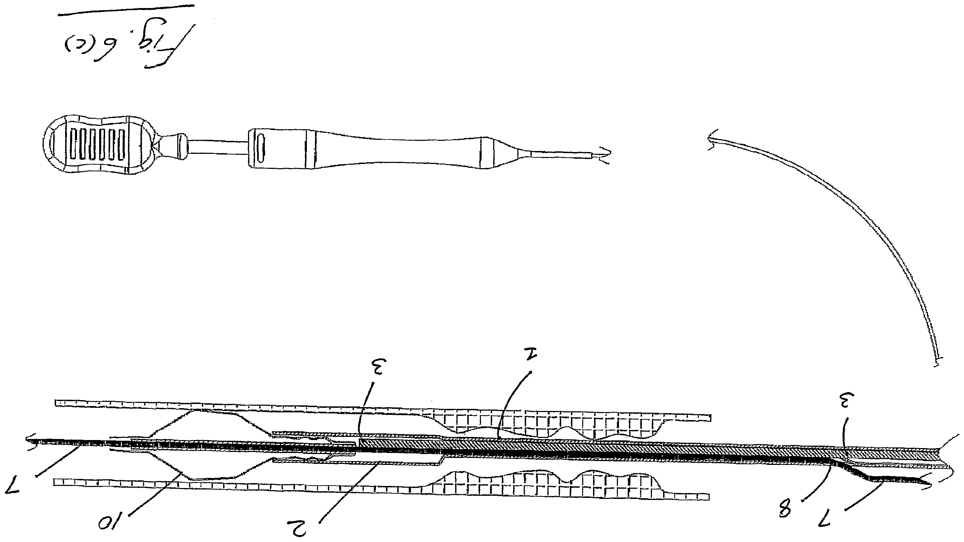

Deployment of the filter 10 is shown in Fig. 6c. The schematic captures the deployment at a stage where the filter 10 is partially deployed from catheter pod 2. The solid inner pusher rod 3 is engaged with the most proximal portion of the filter 10. The abutment surface 9 defines the plane of engagement with the proximal end of the filter 10 which allows the required forces to be transmitted between the solid pusher rod 3 and the filter 10 to enable filter delivery. It should be noted that the solid pusher rod 3 is a very effective force transmitter and may have other applications where a force is required to deploy a medical device from a reception space or indeed where it is necessary to retrieve a device into a reception space.

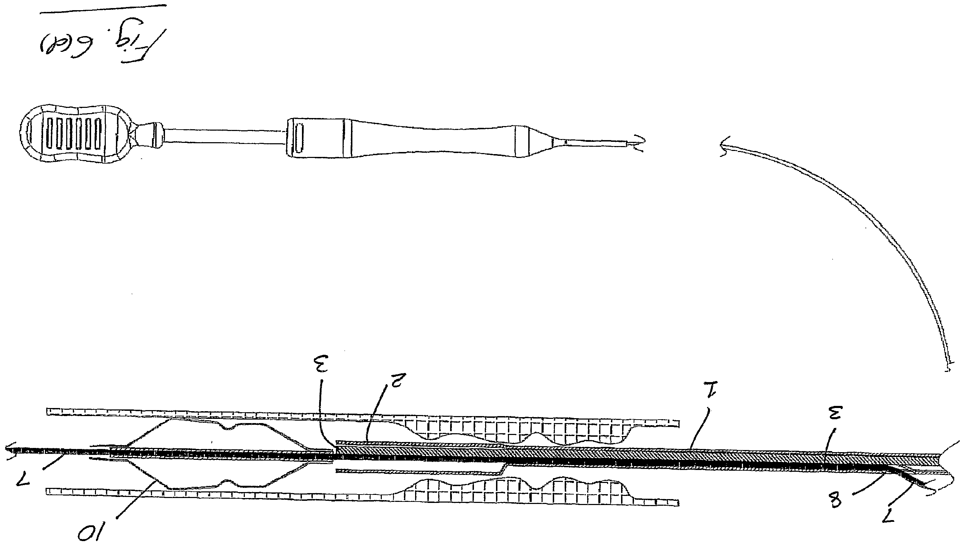

Fig. 6d shows the filter 10 after complete deployment at the intended vascular site with the delivery catheter shown proximally awaiting removal from the vasculature. Fig. 4e shows a filter deployed on a guidewire distal to a lesion site with the delivery catheter removed from the vasculature.

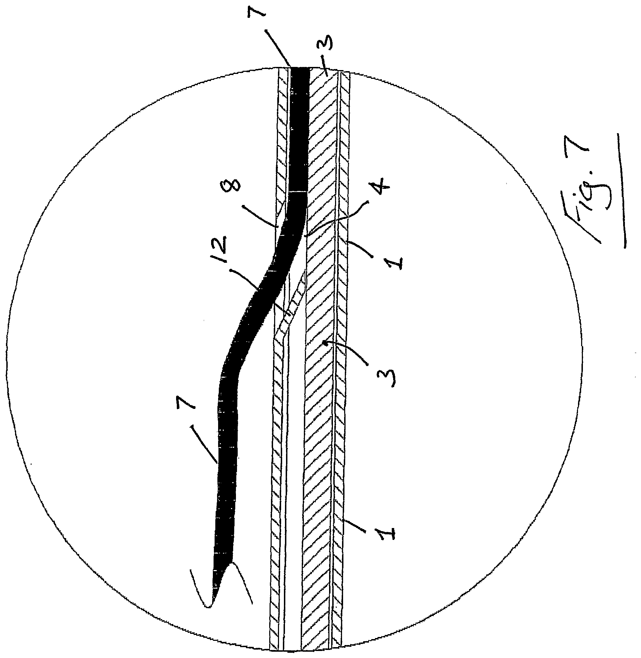

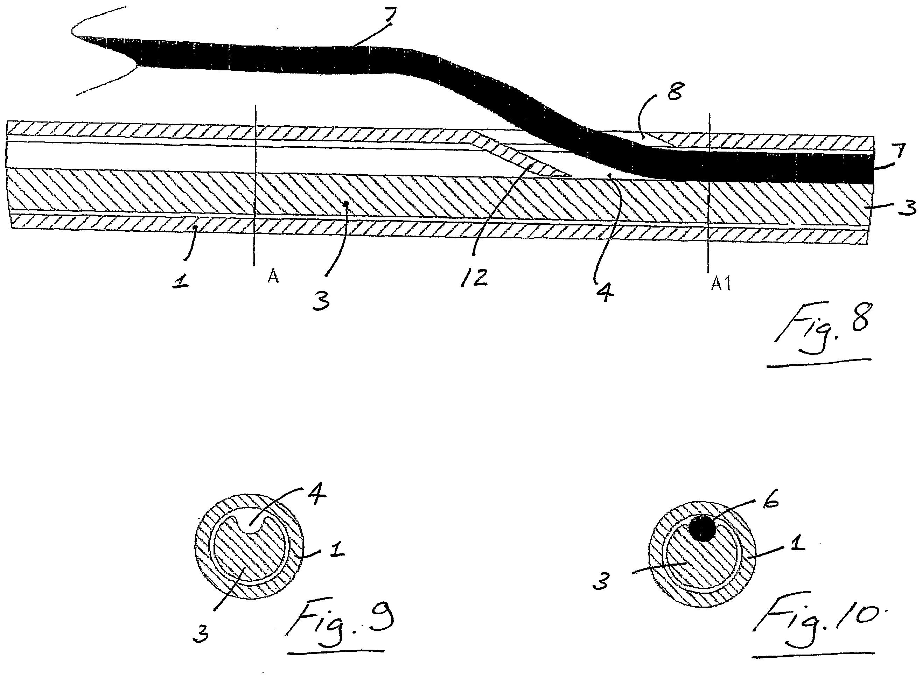

The catheter in the region of the rapid exchange exit port is shown in greater detail in cross-section in Figs. 7 to 10. The guidewire 7 is shown alongside the guidewire surface path 4 distal to the exit port 8. A ramp feature 12 is shown which directs the guidewire away from the guidewire surface path and through the exit port in the outer catheter 1.

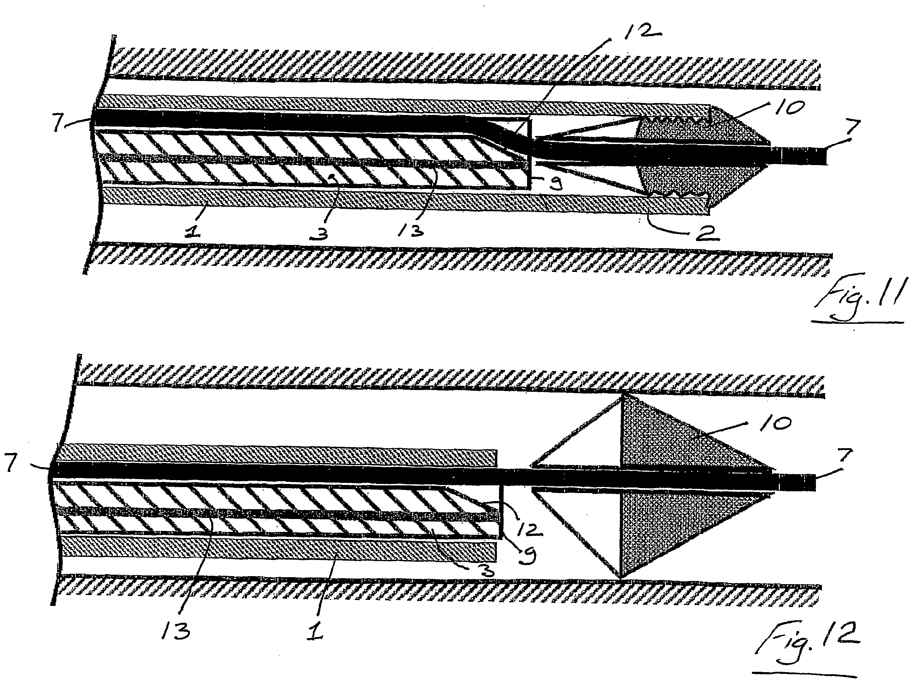

Another configuration of filter delivery system is shown in Fig. 11. In this embodiment the distal end of the shaft 1 comprises the filter reception space 2. It will be noted in this embodiment that there is no off set to the pod 2. Instead, the guidewire 6 is directed to the surface guidewire pathway 4 by means of a ramp 12 at the distal end of the inner member 3. A reinforcement 13 is shown on the centre of area of the cross section of the inner member 3. The reinforcement 13 provides push transmission for the filter deployment action. In one embodiment the reinforcement 13 is manufactured from a metal. In another the reinforcement 13 contains fibre reinforcement. Preferred metals include iron, nickel, chromium, molybdenum, vanadium, titanium or alloys containing one or more of the above. Stainless steels are the most preferred metallic reinforcements. Fibre reinforcements include Kevlar, glass, graphite, carbon, polymer fibres and mixtures of the above. The reinforcement may also have sensory capabilities inbuilt to increase functionality.

Fig. 12 shows the deployed configuration of the system of 11. The inner member 3 has an abutment surface 9 at its distal end that engages the filter for deployment.

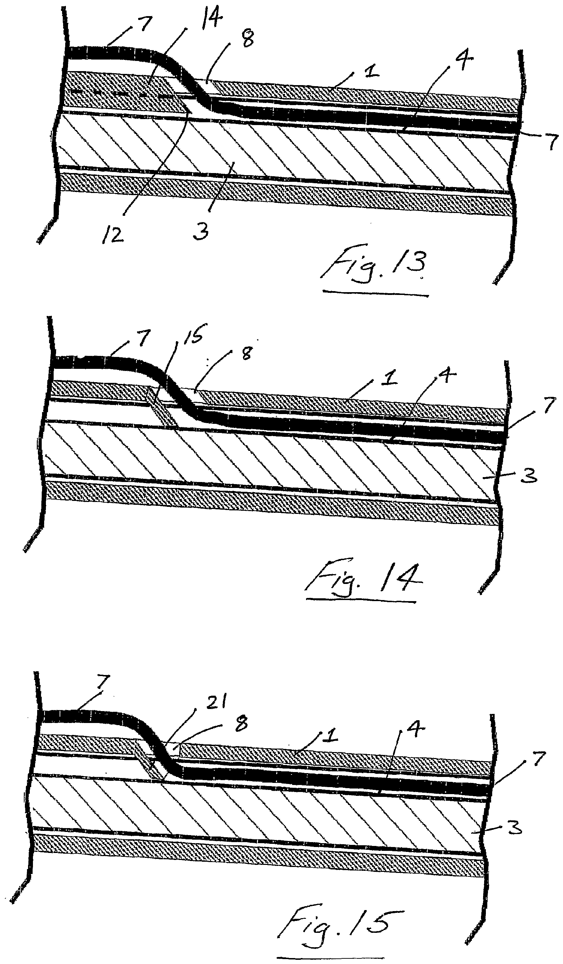

Fig. 13 shows an alternative means for allowing the guidewire 6 to be directed from its side by side contact with the guidewire surface path 4 and exit the outer catheter 1 via the exit port 8. This is facilitated by means of a keying arrangement 14 which is provided on the inner surface of the outer catheter 1 and which engages a surface pathway proximal to the exit port 8. Another feature of the key 14 is that it engages the surface guidewire pathway 4 and prevents relative rotational movement of the inner and outer. The key 14 may also be used to provide an exit ramp 12 at the exit port 8.

Fig. 14 shows an inwardly directed tongue 15 on the outer catheter 1. The tongue 15 engages a surface pathway proximal to the exit port 8. The tongue 15 may also be used to provide an exit ramp at the exit port 8. In Fig. 5 there is illustrated a tongue 21 which may be used to provide an exit ramp and is of a generally cylindrical aspect.

In yet another arrangement the keying function is located in the handle area. This system has the advantage that the user cannot attempt to rotate one element relative to the others and is preferred for this reason. Indeed more than one keying feature can be used simultaneously.

An important feature of all these systems is that they do not inhibit longitudinal movement of the two members.

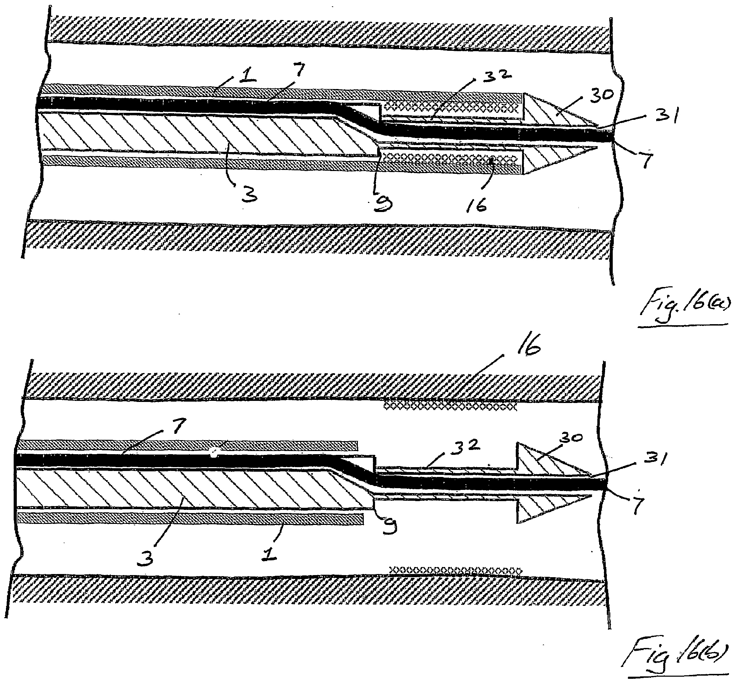

The rapid exchange catheter technology of this invention may be configured as a stent delivery system as illustrated in Figs. 16(a) and 16(b). A self expanding stent 16 is restrained in a reception space at the distal end of the catheter shaft 1. The inner shaft 3 is of solid cross section and contains a surface guidewire pathway 4. The distal end 9 of the inner shaft 3 abuts the proximal end of the stent 16. A tip section 30 is attached to the distal end of the inner shaft. The tip 30 provides a pathway 31 for the guidewire 7 through the body of the stent 16 and guides the guidewire 6 to the surface guidewire pathway of the inner member. The tip 30 further provides a smooth transition between the guidewire 7 and the catheter at its distal end. It will be appreciated that the inner member 3 of this configuration might also be advantageously reinforced to provide push.

A further advantage of this invention is the possibility of using solid markerbands. Because the inner member has no lumens it is possible to attach solid marker bands to the distal end of the shaft ends. This provides exceptionally high levels of visibility of the marker bands.

The stent delivery system of Fig. 16a is shown in the deployed configuration in Fig. 16b. The outer shaft is shown in the retracted position relative to the inner member 3 and the stent 16 deployed in apposition with the vessel 17.

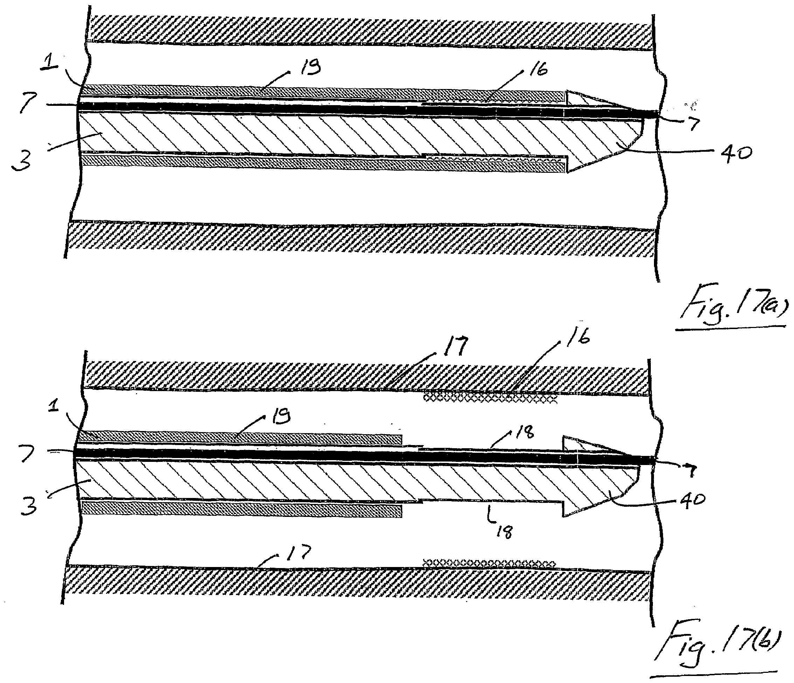

An alternative stent delivery configuration is shown in Figs. 17a and 17b. In this embodiment the inner member 3 comprises a solid shaft with a surface guidewire pathway 4. The solid shaft 3 continues distally to provide a distal tip 40 and contains a recess 18 in the region of the stent 16. The guidewire surface 4 may be in direct contact with the inner surface of elements of the stent 16 as the stent partially defines the guidewire pathway. The stent delivery system is shown in the deployed configuration in Fig. 17b. An outer shaft 19 is shown in the retracted position relative to the inner member 3 and the stent 16 is in apposition with a vessel 17.

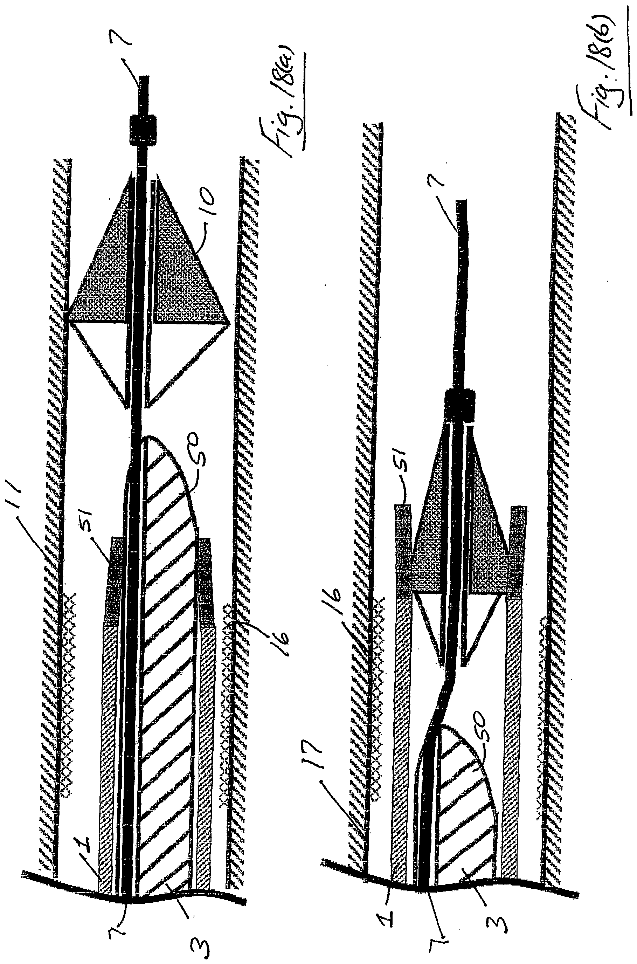

Referring to Figs. 18a and 18b the rapid exchange technology of this invention is illustrated configured as a retrieval system. In this embodiment a solid inner member 3 has a tip 50 that provides a centring function at its distal end. In doing so it provides a smooth transition for crossing stents and lesions. The guidewire 7 is offset relative to the main shaft 1. This has an important advantage compared to conventional retrieval systems in that it can better negotiate stented lesions. Crossing stented lesions is one of the most important design functions of filter retrieval systems. With the trend towards segmented stents crossing implanted stents is becoming increasingly difficult. Segmented stents present a particular problem in that crowns can project into the lumen of the vessel. These crowns can easily snag on a passing retrieval catheter. It can be very difficult to free a retrieval system from such a snag if the vessel is curved as lateral forces continue to push the system onto the crown. This problem can be overcome per this invention due to the eccentric nature of the catheter. By simply torqueing the entire catheter the eccentric tip 50 will relieve a snag and allow crossing by providing a slight lateral movement.

The retrieval system of Fig. 18a is shown with the filter 10 retrieved in Fig. 18b with the filter 10 retrieved into the reception space 2 at the distal end. The distal end of the retrieval catheter is shown with an expansile tip 51. In one embodiment the distal end 50 of the inner shaft 3 is made of a soft material and bends and deflects easily. This allows the tip 51 to deform slightly during retrieval so as to provide good guidewire movement.

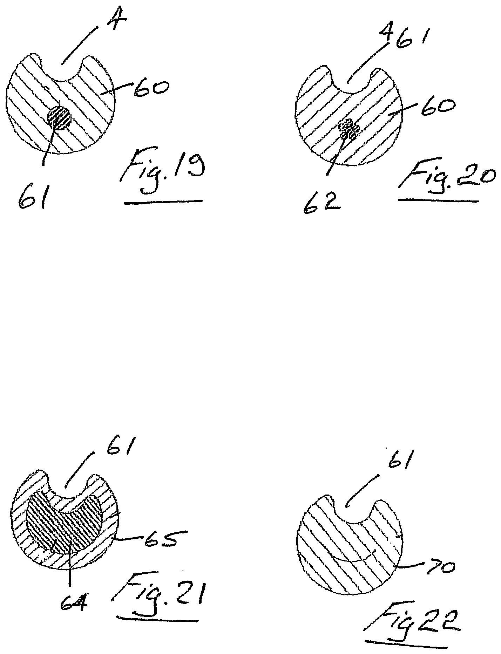

Fig. 19 is a cross-sectional view of a solid inner pusher 60 containing a solid reinforcing wire 61 at its neutral axis. The pusher 60 has a guidewire pathway 4. Because the guidewire has been moved from the central axis location it is now possible to incorporate reinforcing wires or fibers or members in that location. Reinforcements work particularly well with this invention as they can be placed very close to the neutral axis of the inner member irrespective of its plane of bending. Tubular elements require that reinforcements be placed in the walls of the tube. When push properties are desired metallic reinforcements such as wires are desirable. Solid wires or bundles of wires are excellent for transmitting push. Braided systems can be used to optimize torque.

Which ever reinforcement is employed it is desirable that it be placed as close to the center of area of the cross section of the member as possible. Where it is not placed exactly on the center of area it should be centered over the center of area. It should be noted that the center of area is not the same as the central axis of the member. The presence of the surface guidewire pathway 4 shifts the center of area very slightly. Centering reinforcements around the center of area ensures that the properties of the reinforced shaft are very homogenous. Fig. 20 shows a cross-sectional view of a solid inner pusher 60 containing a multifilament wire 62 at its neutral axis. Fig. 21 shows a co-extruded solid pusher with a solid inner of material 64 with a solid outer of material 65. The relative amounts of materials 64 or 65 can be varied to tailor the stiffness and frictional characteristics required by the pusher. The co-extrusions could also me multi-layered or include reinforcement.

Fig. 22 shows a solid inner member 70 that has been manufactured from a tube. The solid member 70 is thus formed by placing a tube in a heated die of the appropriate form and pressure is applied so as to reform the material to the cross section of Fig. 22.

## Claims

1. An intravascular system for the delivery and/or retrieval of a medical device (10, 16) comprising:
an intravascular catheter including an elongate shaft (1);
the elongate shaft (1) having a proximal end, a distal end and a wall defining a shaft lumen; and
an inner member (3) extending through the shaft lumen;
**characterised in that** the inner member is a solid rod member (3),
**in that** the solid rod member (3) has a surface pathway (4) defined by a longitudinally extending partially open portion of the surface of the solid rod member (3) and extending along the length of the solid rod member (3) to direct a guidewire (7) from a distal guidewire port to a proximal guidewire port (8).

2. A system as claimed in claim 1 wherein the proximal guidewire port (8) is positioned a substantial distance distal of the proximal end of the shaft.

3. A system as claimed in claim 1 or 2 wherein the guidewire pathway (4) is offset from a longitudinal axis of at least a portion of the elongate shaft (1).

4. A system as claimed in any of claims 1 to 3 wherein the guidewire pathway (4) is partially defined by an inner surface of the elongate shaft (1).

5. A system as claimed in any of claims 1 to 4 wherein the solid member (3) is slideably translatable relative to the elongate shaft (1).

6. A system as claimed in any of claims 1 to 5 wherein the solid member (3) is slideably translatable relative to a guidewire (7).

7. A system as claimed in any of claims 1 to 6 wherein the catheter comprises a reception space (2) at the distal end of the shaft (1).

8. A system as claimed in claim 7 wherein the reception space (2) is offset relative to the shaft (1) of the catheter, the reception space may be a pod (2), the pod (2) may be thin walled, the pod (2) may be reinforced.

9. A system as claimed in any of claims 1 to 8 wherein the inner solid member (3) is reinforced.

10. A system as claimed in claim 9 wherein the reinforcement (13, 62) is a metal, the metal may be at least partially comprised of one or more of titanium, vanadium, molybdenum, iron, chromium and nickel.

11. A system as claimed in claim 9 or 10 wherein the reinforcemen (13, 62) comprises at least one wire which may be of stainless steel.

12. A system as claimed in claim 9 wherein the reinforcement (13, 62) is a fiber, the fiber may be glass, Kevlar, graphite, carbon or a polymeric fiber.

13. A system as claimed in any of claims 9 to 12 wherein the reinforcement (13, 62) is disposed coaxial to the center of area of the solid member (3).

14. A system as claimed in any of claims 1 to 13 wherein the solid member (3) comprises an abutment surface (9) at its distal end, the abutment surface (9) may be engagable with a medical device (10, 16) for deployment of the device.

15. A system as claimed in any of claims 1 to 14 wherein a tip (30/40) is provided at the distal end of the inner member (3), the tip (30/40) may be integral with the inner member (3), the tip (30/40) may provide a smooth transition between the guidewire (7) and the catheter shaft.

16. A system as claimed in claim 15 wherein the tip (30/40) defines a pathway through the medical device.

17. A system as claimed in claim 15 or 16 wherein the tip (30/40) is of a soft atraumatic material.

18. A system as claimed in any of claims 1 to 17 wherein the lumen of a medical device in the reception space is interfaced with the guidewire surface pathway (4) of the solid member (3).

19. A system as claimed in claim 18 wherein the interface comprises a ramp feature.

20. A system as claimed in claim 18 wherein the interface comprises a portion of a funnel.

21. A system as claimed in any preceding claim wherein the cross sectional area of the inner member (3) added to the cross sectional area of the guidewire (7) comprise substantially the cross sectional area of the inner lumen of the shaft (1).

22. A system as claimed in any of claims 1 to 20 wherein a marker band is provided at the distal end of the inner solid roof (3), the marker band may be attached to the inner shaft, the marker band may be solid in construction.

23. A system as claimed in any of claims 1 to 22 wherein the solid member (3) is rotationally fixed relative to the elongate shaft (1).

24. A system as claimed in claim 23 wherein the system comprises a key (14) to prevent rotational movement.

25. A system as claimed in claim 24 wherein the key (14) is provided in a portion of the outer shaft (1).

26. A system as claimed in claim 24 wherein the key (14) is provided at the guidewire exit port, a ramp may be provided at the exit port and the ramp defines the key.

27. A system as claimed in claim 24 wherein the key is provided in a handle portion of the shaft.

28. A system as claimed in any of claims 1 to 27 wherein the guidewire exit port comprises an opening (8) in the wall of the elongate shaft (1).

29. A system as claimed in claim 28 comprising a ramp (12) for guiding a guidewire (7) to the exit port, the ramp (12) may comprise a tongue-like segment (12) extending inwardly from the exit port, the tongue (12) may extend into the surface guidewire pathway (4) in the inner member (3), the tongue (12) may substantially fill the cross section of the surface guidewire pathway (4).

30. A system as claimed in claim 28 or 29 wherein the ramp surface has a cylindrical aspect.

31. A system as claimed in any of claims 15 to 30 wherein the tip is offset relative to the guidewire.

32. A system as claimed in any of claims 1 to 31 wherein the exit port is sized so as to provide clearance to the guidewire.

33. A system as claimed in any of claims 1 to 32 wherein the guidewire exit port is generally angulated in the direction of guidewire movement.

34. A system as claimed in any of claims 1 to 33 wherein the catheter is a filter delivery catheter, in this case the shaft (1) may have a distal pod defining a reception space for a filter (10).

35. A system as claimed in any of claims 1 to 33 wherein the catheter is a stent delivery catheter, in this case the shaft (1) may have a reception space at the distal end for reception of a stent (16).

36. A system as claimed in claim 35 wherein the inner member (3) extends through a lumen defined by the stent (16), the inner member (3) may terminate in a distal tip.

37. A system as claimed in any of claims 1 to 33 wherein the catheter is a filter retrieval catheter, in this case the shaft (1) may have a distal reception space for reception of a retrieval filter (10).

38. A system as claimed in claim 37 wherein the inner member (3) extends through the reception space during positioning of the catheter for filter retrieval, the inner member (3) may project distally of the distal end of the shaft (1) to provide centering.

## Patentansprüche

1. Intravaskuläres System zum Zuführen und/oder Zurückholen einer medizinischen Vorrichtung (10, 16), umfassend:
einen intravaskulären Katheter, umfassend einen langgestreckten Schaft (1);
wobei der langgestreckte Schaft (1) ein proximales Ende, ein distales Ende und eine ein Schaftlumen definierende Wand aufweist; und
ein inneres Element (3), das sich durch das Schaftlumen erstreckt;
**dadurch gekennzeichnet, dass** es sich bei dem inneren Element um ein massives Stangenelement (3) handelt,
dass das massive Stangenelement (3) eine Oberflächenbahn (4) aufweist, die von einem sich längs erstreckenden, teilweise offenen Abschnitt der Oberfläche des massiven Stangenelements (3) definiert wird und sich entlang der Länge des massiven Stangenelements (3) erstreckt, um einen Führungsdraht (7) von einer distalen Führungsdrahtöffnung zu einer proximalen Führungsdrahtöffnung (8) zu lenken.

2. System nach Anspruch 1, wobei die proximale Führungsdrahtöffnung (8) in einem erheblichen Abstand distal des proximalen Endes des Schafts positioniert ist.

3. System nach Anspruch 1 oder 2, wobei die Führungsdrahtbahn (4) von einer Längsachse mindestens eines Abschnitts des langgestreckten Schafts (1) versetzt ist.

4. System nach einem der Ansprüche 1 bis 3, wobei die Führungsdrahtbahn (4) teilweise von einer inneren Oberfläche des langgestreckten Schafts (1) definiert wird.

5. System nach einem der Ansprüche 1 bis 4, wobei das massive Element (3) relativ zu dem langgestreckten Schaft (1) gleitend verschiebbar ist.

6. System nach einem der Ansprüche 1 bis 5, wobei das massive Element (3) relativ zu einem Führungsdraht (7) gleitend verschiebbar ist.

7. System nach einem der Ansprüche 1 bis 6, wobei der Katheter einen Aufnahmeraum (2) am distalen Ende des Schafts (1) umfasst.

8. System nach Anspruch 7, wobei der Aufnahmeraum (2) relativ zu dem Schaft (1) des Katheters versetzt ist, es sich bei dem Aufnahmeraum um eine Kapsel (2) handeln kann, die Kapsel (2) dünnwandig sein kann, wobei die Kapsel (2) verstärkt sein kann.

9. System nach einem der Ansprüche 1 bis 8, wobei das innere massive Element (3) verstärkt ist.

10. System nach Anspruch 9, wobei es sich bei der Verstärkung (13, 62) um ein Metall handelt, das Metall mindestens teilweise aus einem oder mehr von Titan, Vanadium, Molybdän, Eisen, Chrom und Nickel bestehen kann.

11. System nach Anspruch 9 oder 10, wobei die Verstärkung (13, 62) mindestens einen Draht umfasst, der aus Edelstahl sein kann.

12. System nach Anspruch 9, wobei es sich bei der Verstärkung (13, 62) um eine Faser handelt, es sich bei der Faser um Glas, Kevlar, Graphit, Kohlenstoff oder eine Polymerfaser handeln kann.

13. System nach einem der Ansprüche 9 bis 12, wobei die Verstärkung (13, 62) koaxial zur Mitte der Fläche des massiven Elements (3) angeordnet ist.

14. System nach einem der Ansprüche 1 bis 13, wobei das massive Element (3) eine Anlagefläche (9) an seinem distalen Ende umfasst, die Anlagefläche (9) mit einer medizinischen Vorrichtung (10, 16) kuppelbar sein kann, um die Vorrichtung auszufahren.

15. System nach einem der Ansprüche 1 bis 14, wobei eine Spitze (30/40) am distalen Ende des inneren Elements (3) bereitgestellt ist, die Spitze (30/40) mit dem inneren Element (3) integriert sein kann, die Spitze (30/40) einen glatten Übergang zwischen dem Führungsdraht (7) und dem Katheterschaft bereitstellen kann.

16. System nach Anspruch 15, wobei die Spitze (30/40) eine Bahn durch die medizinische Vorrichtung definiert.

17. System nach Anspruch 15 oder 16, wobei die Spitze (30/40) aus einem weichen atraumatischen Material ist.

18. System nach einem der Ansprüche 1 bis 17, wobei das Lumen einer medizinischen Vorrichtung in dem Aufnahmeraum mit der Führungsdraht-Oberflächenbahn (4) des massiven Elements (3) in Verbindung steht.

19. System nach Anspruch 18, wobei die Verbindung ein Rampenmerkmal umfasst.

20. System nach Anspruch 18, wobei die Verbindung einen Abschnitt eines Trichters umfasst.

21. System nach einem der vorangehenden Ansprüche, wobei die Querschnittsfläche des inneren Elements (3) und die Querschnittsfläche des Führungsdrahts (7) zueinander addiert im Wesentlichen die Querschnittsfläche des inneren Lumen des Schafts (1) umfassen.

22. System nach einem der Ansprüche 1 bis 20, wobei ein Markierungsband am distalen Ende des inneren massiven Dachs (3) bereitgestellt ist, wobei das Markierungsband an dem inneren Schaft angebracht sein kann, das Markierungsband einen massiven Aufbau aufweisen kann.

23. System nach einem der Ansprüche 1 bis 22, wobei das massive Element (3) relativ zu dem langgestreckten Schaft (1) drehfest ist.

24. System nach Anspruch 23, wobei das System einen Längskeil (14) umfasst, um Drehbewegung zu verhindern.

25. System nach Anspruch 24, wobei der Längskeil (14) in einem Abschnitt des äußeren Schafts (1) bereitgestellt ist.

26. System nach Anspruch 24, wobei der Längskeil (14) an der Führungsdraht-Austrittsöffnung bereitgestellt ist, eine Rampe an der Austrittsöffnung bereitgestellt sein kann und die Rampe den Längskeil definiert.

27. System nach Anspruch 24, wobei der Längskeil in einem Griffabschnitt des Schafts bereitgestellt ist.

28. System nach einem der Ansprüche 1 bis 27, wobei die Führungsdraht-Austrittsöffnung eine Aussparung (8) in der Wand des langgestreckten Schafts (1) umfasst.

29. System nach Anspruch 28, umfassend eine Rampe (12) zum Führen eines Führungsdrahts (7) zu der Austrittsöffnung, wobei die Rampe (12) ein zungenartiges Segment (12) umfassen kann, das sich von der Austrittsöffnung nach innen erstrecken kann, sich die Zunge (12) in die Oberflächen-Führungsdrahtbahn (4) in dem inneren Element (3) erstrecken kann, die Zunge (12) im Wesentlichen den Querschnitt der Oberflächen-Führungsdrahtbahn (4) füllen kann.

30. System nach Anspruch 28 oder 29, wobei die Rampenoberfläche eine zylindrische Gestalt aufweist.

31. System nach einem der Ansprüche 15 bis 30, wobei die Spitze relativ zu dem Führungsdraht versetzt ist.

32. System nach einem der Ansprüche 1 bis 31, wobei die Austrittsöffnung derart bemessen ist, dass sie Freiraum für den Führungsdraht bereitstellt.

33. System nach einem der Ansprüche 1 bis 32, wobei die Führungsdraht-Austrittsöffnung allgemein in der Richtung der Führungsdrahtbewegung gewinkelt ist.

34. System nach einem der Ansprüche 1 bis 33, wobei es sich bei dem Katheter um einen Filterzuführungskatheter handelt, wobei in diesem Fall der Schaft (1) eine distale Kapsel aufweisen kann, die einen Aufnahmeraum für einen Filter (10) definiert.

35. System nach einem der Ansprüche 1 bis 33, wobei es sich bei dem Katheter um einen Stentzuführungskatheter handelt, wobei in diesem Fall der Schaft (1) einen Aufnahmeraum am distalen Ende zum Aufnehmen eines Stents (16) aufweisen kann.

36. System nach Anspruch 35, wobei sich das innere Element (3) durch ein von dem Stent (16) definiertes Lumen erstreckt, wobei das innere Element (3) in einer distalen Spitze enden kann.

37. System nach einem der Ansprüche 1 bis 33, wobei es sich bei dem Katheter um einen Filterrückholkatheter handelt, wobei in diesem Fall der Schaft (1) einen distalen Aufnahmeraum zum Aufnehmen eines Rückholfilters (10) aufweisen kann.

38. System nach Anspruch 37, wobei sich das innere Element (3) während des Positionierens des Katheters zur Filterrückholung durch den Aufnahmeraum erstreckt, wobei das innere Element (3) distal des distalen Endes des Schafts (1) vorstehen kann, um Zentrierung bereitzustellen.

## Revendications

1. Système intravasculaire destiné à la mise en place et/ou au retrait d'un dispositif médical (10, 16) comportant :
un cathéter intravasculaire comprenant une tige allongée (1) ;
la tige allongée (1) ayant une extrémité proximale, une extrémité distale et une paroi définissant une lumière de tige ; et
un élément intérieur (3) s'étendant au travers de la lumière de tige ;
**caractérisé en ce que** l'élément intérieur est un élément formant tige pleine (3),
**en ce que** l'élément formant tige pleine (3) a une voie de passage de surface (4) définie par une partie partiellement ouverte s'étendant dans le sens longitudinal de la surface de l'élément formant tige pleine (3) et s'étendant sur toute la longueur de l'élément formant tige pleine (3) pour diriger un fil-guide (7) depuis un orifice de fil-guide distal jusqu'à un orifice de fil-guide proximal (8).

2. Système selon la revendication 1, dans lequel l'orifice de fil-guide proximal (8) est positionné à une importante distance distale par rapport à l'extrémité proximale de la tige.

3. Système selon la revendication 1 ou la revendication 2, dans lequel la voie de passage de fil-guide (4) est décalée par rapport à un axe longitudinal d'au moins une partie de la tige allongée (1).

4. Système selon l'une quelconque des revendications 1 à 3, dans lequel la voie de passage de fil-guide (4) est partiellement définie par une surface intérieure de la tige allongée (1).

5. Système selon l'une quelconque des revendications 1 à 4, dans lequel l'élément plein (3) est en mesure de translater de manière coulissante par rapport à la tige allongée (1).

6. Système selon l'une quelconque des revendications 1 à 5, dans lequel l'élément plein (3) est en mesure de translater de manière coulissante par rapport à un fil-guide (7).

7. Système selon l'une quelconque des revendications 1 à 6, dans lequel le cathéter comporte un espace de réception (2) au niveau de l'extrémité distale de la tige (1).

8. Système selon la revendication 7, dans lequel l'espace de réception (2) est décalé par rapport à la tige (1) du cathéter, l'espace de réception peut être une gaine (2), la gaine (2) peut être à paroi mince, la gaine (2) peut être renforcée.

9. Système selon l'une quelconque des revendications 1 à 8, dans lequel l'élément plein intérieur (3) est renforcé.

10. Système selon la revendication 9, dans lequel le renfort (13, 62) est un métal, le métal peut être au moins partiellement constitué par un ou plusieurs parmi du titane, du vanadium, du molybdène, du fer, du chrome et du nickel.

11. Système selon la revendication 9 ou la revendication 10, dans lequel le renfort (13, 62) comprend au moins un fil qui peut être en acier inoxydable.

12. Système selon la revendication 9, dans lequel le renfort (13, 62) est une fibre, la fibre peut être du verre, de la fibre aramide, du graphite, du carbone ou une fibre de plastique.

13. Système selon l'une quelconque des revendications 9 à 12, dans lequel le renfort (13, 62) est disposé de manière coaxiale par rapport au centre de la surface de l'élément plein (3).

14. Système selon l'une quelconque des revendications 1 à 13, dans lequel l'élément plein (3) comporte une surface de mise en butée (9) au niveau de son extrémité distale, la surface de mise en butée (9) peut être mise en prise avec un dispositif médical (10, 16) à des fins de déploiement du dispositif.

15. Système selon l'une quelconque des revendications 1 à 14, dans lequel un embout (30/40) est mis en oeuvre au niveau de l'extrémité distale de l'élément intérieur (3), l'embout (30/40) peut faire partie intégrante de l'élément intérieur (3), l'embout (30/40) peut procurer une transition sans à-coups entre le fil-guide (7) et la tige de cathéter.

16. Système selon la revendication 15, dans lequel l'embout (30/40) définit une voie de passage au travers du dispositif médical.

17. Système selon la revendication 15 ou la revendication 16, dans lequel l'embout (30/40) est constitué d'un matériau atraumatique flexible.

18. Système selon l'une quelconque des revendications 1 à 17, dans lequel la lumière d'un dispositif médical dans l'espace de réception a une interface avec la voie de passage de surface de fil-guide (4) de l'élément plein (3).

19. Système selon la revendication 18, dans lequel l'interface comporte un dispositif formant rampe.

20. Système selon la revendication 18, dans lequel l'interface comporte une partie d'un entonnoir.

21. Système selon l'une quelconque des revendications précédentes, dans lequel la section transversale de l'élément intérieur (3) ajoutée à la section transversale du fil-guide (7) constitue sensiblement la section transversale de la lumière intérieure de la tige (1).

22. Système selon l'une quelconque des revendications 1 à 20, dans lequel une bande de marqueur est mise en oeuvre au niveau de l'extrémité distale du toit plein intérieur (3), la bande de marqueur peut être attachée à la tige intérieure, la bande de marqueur peut être d'une construction pleine.

23. Système selon l'une quelconque des revendications 1 à 22, dans lequel l'élément plein (3) est fixé de manière rotative par rapport à la tige allongée (1).

24. Système selon la revendication 23, dans lequel le système comporte une clavette (14) pour empêcher tout mouvement de rotation.

25. Système selon la revendication 24, dans lequel la clavette (14) est mise en oeuvre dans une partie de la tige extérieure (1).

26. Système selon la revendication 24, dans lequel la clavette (14) est mise en oeuvre au niveau de l'orifice de sortie de fil-guide, une rampe peut être mise en oeuvre au niveau de l'orifice de sortie et la rampe définit la clavette.

27. Système selon la revendication 24, dans lequel la clavette est mise en oeuvre dans une partie manche de la tige.

28. Système selon l'une quelconque des revendications 1 à 27, dans lequel l'orifice de sortie de fil-guide comporte une ouverture (8) dans la paroi de la tige allongée (1).

29. Système selon la revendication 28, comportant une rampe (12) servant à des fins de guidage d'un fil-guide (7) jusqu'à l'orifice de sortie, la rampe (12) peut comporter un segment similaire à une languette (12) s'étendant vers l'intérieur depuis l'orifice de sortie, la languette (12) peut s'étendre jusque dans la voie de passage de fil-guide de surface (4) dans l'élément intérieur (3), la languette (12) peut sensiblement remplir la section transversale de la voie de passage de fil-guide de surface (4).

30. Système selon la revendication 28 ou la revendication 29, dans lequel la surface de la rampe a un aspect cylindrique.

31. Système selon l'une quelconque des revendications 15 à 30, dans lequel l'embout est décalé par rapport au fil-guide.

32. Système selon l'une quelconque des revendications 1 à 31, dans lequel l'orifice de sortie est dimensionné de manière à fournir un espace libre au fil-guide.

33. Système selon l'une quelconque des revendications 1 à 32, dans lequel l'orifice de sortie de fil-guide est généralement angulé dans la direction du mouvement du fil-guide.

34. Système selon l'une quelconque des revendications 1 à 33, dans lequel le cathéter est un cathéter de mise en place de filtre, dans ce cas la tige (1) peut avoir une gaine distale définissant un espace de réception pour un filtre (10).

35. Système selon l'une quelconque des revendications 1 à 33, dans lequel le cathéter est un cathéter de mise en place d'endoprothèse vasculaire, dans ce cas la tige (1) peut avoir un espace de réception au niveau de l'extrémité distale à des fins de réception d'une endoprothèse vasculaire (16).

36. Système selon la revendication 35, dans lequel l'élément intérieur (3) s'étend au travers d'une lumière définie par l'endoprothèse vasculaire (16), l'élément intérieur (3) peut se terminer dans un embout distal.

37. Système selon l'une quelconque des revendications 1 à 33, dans lequel le cathéter est un cathéter de retrait de filtre, dans ce cas la tige (1) peut avoir un espace de réception distal à des fins de réception d'un filtre de retrait (10).

38. Système selon la revendication 37, dans lequel l'élément intérieur (3) s'étend au travers de l'espace de réception au cours du positionnement du cathéter à des fins de retrait du filtre, l'élément intérieur (3) peut faire saillie de manière distale par rapport à l'extrémité distale de la tige (1) pour fournir un centrage.
